(19) Europäisches Patentamt
European Patent Office
Office européen des brevets

(11) Veröffentlichungsnummer: **0 163 236**
**B1**

(12) **EUROPÄISCHE PATENTSCHRIFT**

(45) Veröffentlichungstag der Patentschrift: **25.04.90**

(21) Anmeldenummer: **85106199.4**

(22) Anmeldetag: **21.05.85**

(51) Int. Cl.$^5$: **C 07 D 263/58**, C 07 D 277/82, A 01 N 43/76, A 01 N 43/78

(54) **Benzoxazolyl- und Benzthiazolyl-aminosäuren, ihre Herstellung und Verwendung im Pflanzenschutz.**

(30) Priorität: **29.05.84 DE 3419994**

(43) Veröffentlichungstag der Anmeldung:
**04.12.85 Patentblatt 85/49**

(45) Bekanntmachung des Hinweises auf die Patenterteilung:
**25.04.90 Patentblatt 90/17**

(84) Benannte Vertragsstaaten:
**AT BE CH DE FR GB IT LI NL SE**

(56) Entgegenhaltungen:
**JOURNAL OF PHARMACEUTICAL SCIENCES, Band 57, Nr. 10, Oktober 1968, Seiten 1693-1696, Washington, D.C., US; S.B. ADVANI et al.: "Potential antineoplastic agents: N-(2-benzoxazolyl)amino acid esters"**

**HELVETICA CHIMICA ACTA, Band 50, Nr. 4, 31. Mai 1967, Seiten 1084-1086, Basel, CH; H. SUTER et al.: "Studien über Benzthiazole als eventuelle orale Antidiabetica"**

(73) Patentinhaber: **HOECHST AKTIENGESELLSCHAFT**
**Postfach 80 03 20**
**D-6230 Frankfurt am Main 80 (DE)**

(72) Erfinder: **Boesenberg, Heinz, Dr.**
**Am Dachsgraben 4**
**D-6238 Hofheim am Taunus (DE)**
Erfinder: **Mildenberger, Hilmar, Dr.**
**Fasanenstrasse 24**
**D-6238 Kelkheim (Taunus) (DE)**
Erfinder: **Bauer, Klaus, Dr.**
**Kolpingstrasse 7**
**D-6054 Rodgau (DE)**
Erfinder: **Bieringer, Hermann, Dr.**
**Eichenweg 26**
**D-6239 Eppstein/Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 163 236 B1

**Beschreibung**

Die vorliegende Erfindung betrifft die Verwendung von Verbindungen der allgemeinen Formel I

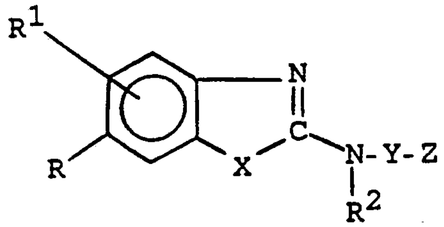

worin

X = O oder S;

R, $R^1$ = Wasserstoff, Halogen, ($C_1$—$C_4$)-Alkyl, ($C_1$—$C_4$)-Alkoxy, CN, $NO_2$ oder $CF_3$;

$R^2$ = Wasserstoff oder ($C_1$—$C_4$)-Alkyl;

Y = geradkettiges oder verzweigtes ($C_1$—$C_8$)-Alkylen, von dem sich 1—3 C-Atome in gerader Kette zwischen dem Amin-Stickstoff und der Gruppe Z befinden; Methylthio-($C_1$—$C_3$)-alkylen, Phenyl-($C_1$—$C_3$)-alkylen oder Phenylen;

Z = 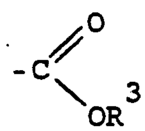 , 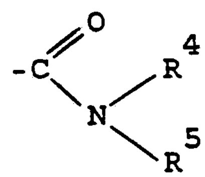 , -CN oder 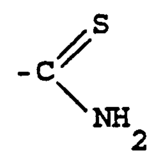 ;

$R^3$ = H, ($C_1$—$C_8$)-Alkyl, das ggf. durch Halogen oder ($C_1$—$C_2$)-Alkoxy substituiert sein kann, ($C_3$—$C_4$)-Alkenyl, ($C_3$—$C_4$)-Alkinyl oder das Kation einer anorganischen oder organischen Base;

$R^4$ = H, ($C_1$—$C_6$)-Alkyl;

$R^5$ = H, ($C_1$—$C_6$)-Alkyl oder Phenyl, das ggf. 1- oder 2-fach durch Halogen, ($C_1$—$C_4$)-Alkyl oder ($C_1$—$C_4$)-Alkoxy substituiert sein kann; oder

$R^4$ und $R^5$ zusammen mit dem Stickstoffatom einen Pyrrolidino-, Piperidino- oder Morpholino-Rest bedeuten, zum Schutz von Nutzpflanzen gegen phytotoxische Wirkungen von Herbiziden.

Als Kationen kommen z.B. die folgenden in Betracht:

$Na^+$ , $K^+$, $NH_4^+$ , 1/2 $Ca^{++}$, $H_3N^+-CH_3$, $H_3N^+(i\text{-}C_3H_7)$, $H_2N^+(CH_3)_2$,

$HN^+(CH_3)_3$, $H_2N^+(n\text{-}C_4H_9)_2$, 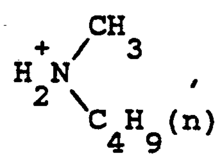 , $H_3N^+-C_6H_5$, 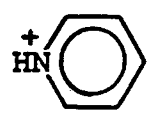 ,

$H_3N^+-CH_2CH_2OH$ oder $HN^+(CH_2CH_2OH)_3$ .

Bevorzugt werden die jenigen Verbindungen der Formel I als pflanzenschützende Mittel verwendet, in denen der Benzoxazolyl- oder Benzthiazolyl-Rest im aromatischen Ring unsubstituiert oder durch Halogen einfach substituiert ist, $R^2$ H oder Methyl; Y $C_1$- oder $C_2$-Alkylen, Z die Gruppe —$COOR^3$ und $R^3$ ($C_1$—$C_8$)Alkyl, ($C_3$—$C_4$)Alkenyl, ($C_3$—$C_4$)Alkinyl oder ein Kation bedeuten.

Einige Verbindungen der allgemeinen Formel I — ausnahmslos solche mit R = $R^1$ = $R^2$ = H — sind in der Literatur beschrieben [Helv. Chim. Acta *50* (1967), 1084—86; J. Pharm. Sc. *57* (1968), 1693—96]. Diese Substanzen wurden in Zusammenhang mit pharmazeutischen Fragestellungen synthetisiert und erwiesen sich hierbei als unwirksam. Die übrigen Verbindungen der Formel I sind jedoch neu.

Gegenstand der Erfindung sind daher auch die Verbindungen der Formel I worin

Y O oder S

R H, $CH_3$ oder Cl

$R^1$ H

$R^2$ H oder $CH_3$

Y $C_1$- oder $C_2$-Alkylen,

Z einen Rest der Formel —$COOR^3$ und $R^3$ ($C_1$—$C_8$)Alkyl, ($C_3$—$C_4$)Alkenyl, ($C_3$—$C_4$)-Alkinyl oder ein

Kation bedeuten, mit der Maßgabe, daß mindestens einer der Reste R und $R^2$ von Wasserstoff verschieden ist, sowie Verfahren zu ihrer Herstellung.

Man erhält die neuen Verbindungen, indem man

a) Verbindungen der Formel

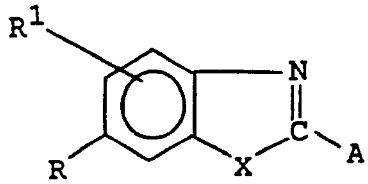

(II)

worin A eine leicht abspaltbare Gruppe ("leaving group") bedeutet, mit Verbindungen der Formel

$$\underset{\displaystyle R^2}{\overset{|}{HN}}—Y—COOR^3 \qquad (III)$$

oder

b) Verbindungen der Formel

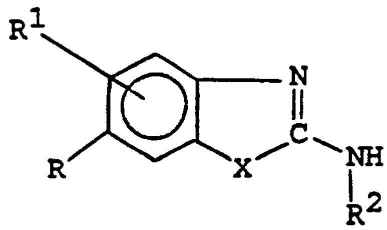

(IV)

mit Verbindungen der Formel

$$Hal—Y—COOR^3 \qquad (V)$$

umsetzt und gewünschtenfalls die erhaltenen Verbindungen in an sich bekannter Weise, z.B. durch Veresterung, Verseifung, Salzbildung, Amidierung, Alkylierung oder Dehydratisierung in andere Verbindungen der Formel I überführt. Zu den Formeln II—V haben R, $R^1$, $R^2$, $R^3$, X und Y die angegebene Bedeutung, jedoch mit der Maßgabe, daß mindestens einer der Reste R, $R^1$ und $R^2$ von Wasserstoff verschieden ist.

*Zu a)* Die Verbindungen der Formel III können in Form der freien Aminosäuren, der Aminosäureester oder deren Hydrohalogeniden, vorzugsweise der Hydrochloride, eingesetzt werden. Als Verbindungen der Formel II verwendet man bevorzugt die Chlor- oder Bromverbindungen und arbeitet dann mit einem Zusatz von halogenwasserstoffbindenden Agentien bei Temperaturen zwischen 20 und 150°C, vorzugsweise bei 50 bis 120°C. Als Lösungsmittel kommen Alkohole wie Methanol, Ethanol, Isopropanol, n-Butanol; Ether wie Diethylether, Diisopropylether, Di-n-butylether, Ethylenglykoldimethylether, Tetrahydrofuran oder 1,4-Dioxan; Ester, wie Essigsäure-ethylester oder Essigsäure-n-butylester; Kohlenwasserstoffe wie Hexan, Benzol, Toluol, Xylol; Halogenkohlenwasserstoffe, wie Dichlormethan, Chloroform, 1,2-Dichlor-ethan oder Chlor-benzol in Betracht. Auch polare Lösungsmittel wie Wasser, Acetonitril, Dimethylformamid, Dimethylsulfoxid, N-Methylpyrrolidon oder Gemische der vorgenannten Lösungsmittel können eingesetzt werden.

Als Halogenwasserstoff-bindende Mittel eignen sich NaOH, KOH, $NaHCO_3$, $K_2CO_3$, ferner tertiäre Amine wie Triethylamin, Pyridin, N,N-Dimethyl-anilin oder ein Überschuß der eingesetzten Aminosäure oder deren Ester.

Statt der Halogenderivate II kann man auch Ausgangsstoffe mit anderen leicht abspaltbaren Gruppen ("leaving groups") in 2-Stellung verwenden. Geeignet für diese Umsetzung sind z.B. Alkylthio-, Alkylsulfinyl- oder Alkylsulfonylgruppen wie der Methylthio-, der Äthylsulfinyl- oder der Methylsulfonyl-Rest. Auch aromatische Abgangsgruppen wie die 4-Toluol-sulfonylgruppe sind brauchbar. Hier arbeitet man bei etwas höheren Temperaturen zwischen 50 und 200°C — vorzugsweise bei 80 bis 150°C — und in Gegenwart eines inerten, höher siedenden Lösungsmittels wie Toluol, Xylol oder Chlorbenzol.

*Zu b):* Die umgekehrte Reaktion von 2-Aminobenzoxazolen oder -benzthiazolen IV mit α-Halogen-carbonsäuren bzw. deren Estern verläuft unter den gleichen Bedingungen wie unter a) beschrieben.

Die erhaltenen Verbindungen der Formel I lassen sich in bekannter Weise in andere Derivate der Formel I überführen. So erhält man durch Verseifung der Ester die freien Säuren oder ihre Salze. Zu den Amiden gelangt man durch Aminolyse der Ester oder durch Umsetzung der freien Säuren nach der Carbonyl-diimidazolidmethode. Durch Veresterung der freien Säuren entstehen weitere Ester.

Entwässerung von Amiden liefert die Nitrile (Z = CN), die wiederum mit $H_2S$ in Thioamide (Z = —$CSNH_2$) überführt werden können. Schließlich können die Verbindungen mit $R^2$ = H gewünschtenfalls am Stickstoff alkyliert werden. All diese Umsetzungen sind dem Fachmann wohlbekannt und bedürfen keiner näheren Erläuterung.

Überraschend wurde gefunden, daß die Verbindungen der Formel I die Eigenschaften haben, phytotoxische Nebenwirkungen von Pflanzenschutzmitteln, insbesondere von Herbiziden, beim Einsatz in Nutzpflanzenkulturen zu vermindern und auszuschalten.

Die erfindungsgemäßen Mittel können also vor, nach oder (vorzugsweise) gleichzeitig mit anderen Herbiziden ausgebracht werden und sind dann in der Lage, eventuelle schädliche Nebenwirkungen dieser Herbizide bei Nutzpflanzen zu antagonisieren, d.h. aufzuheben, ohne ihre erwünschte herbizide Wirksamkeit zu beeinträchtigen. Hierdurch kann das Einsatzgebiet herkömmlicher Pflanzenschutzmittel erheblich vergrößert werden. Solche Verbindungen, die die genannten Eigenschaften besitzen, werden "Antidote" oder "Safener" genannt.

Der Einsatz von Safenern zum Schutze von Nutzpflanzenkulturen gegen Schädigung durch Herbizide ist an sich seit längerem bekannt. Benzoxazolyl-(2) oder Benzthiazolyl-(2)-aminosäuren und ihre Derivate der allgemeinen Formel I sind als Safener bisher jedoch nicht verwendet geworden.

Herbizide, deren phytotoxische Nebenwirkungen mittels Verbindungen der Formel I herabgesetzt werden können, sind z.B. substituierte Phenoxyphenoxy-carbonsäureester sowie Benzoxazolyloxy-, Benzthiazolyloxy- und Benzylphenoxycarbonsäureester, ferner Dimedonoximabkömmlinge. Beispielsweise seien, ohne daß dadurch eine Beschränkung erfolgen soll, folgende Herbizide genannt:

Phenoxycarbonsäureester wie
2-(4-(2,4-Dichlorphenoxy)-phenoxy)-propionsäuremethylester (Diclofop-methyl),
2-(4-(4-Brom-2-chlorphenoxy)-phenoxy)-propionsäuremethylester,
2-(4-Trifluormethylphenoxy)-phenoxy-propionsäuremethylester,
2-(4-(2-Chlor-4-trifluormethylphenoxy)-phenoxy)-propionsäuremethylester,
2-(4-(2,4-Dichlorbenzyl)-phenoxy)-propionsäuremethylester,
2-(4-(4-Trifluormethylphenoxy)-phenoxy)-2-penten-1-carbonsäureethylester,
2-(4-(6-Chlorbenzoxazol-2-yl-oxy)-phenoxy)-propionsäureethylester (Fenoxaprop-ethyl),
2-(4-(6-Chlorbenzthiazol-2-yl-oxy)-phenoxy)-propionsäure-ethylester
Dimedon-Derivate wie
2-(N-Äthoxybutyrimidoyl)-5-(2-ethylthiopropyl)-3-hydroxy-2-cyclohexen-1-on oder
2-(1-Allyloxyiminobutyl)-4-methoxycarbonyl-5,5-dimethyl-3-oxocyclohexanol.

Für die Anwendung können die Verbindungen der Formel I mit üblichen Formulierungshilfsmitteln zu Stäubemitteln, Spritzpulvern, Dispersionen, Emulsionskonzentraten usw. zubereitet werden, die den Wirkstoff in Konzentrationen von 2—80% enthalten und entweder als solche angewendet werden (Stäubemitteln, Pellets) oder vor der Anwendung in einem Lösungsmittel (Wasser) gelöst oder dispergiert werden.

Das Mengenverhältnis Antidot:Herbizid kann innerhalb weiter Grenzen zwischen 0,01 und 10 Teilen Antidot auf 1 Teile Herbizid schwanken. Die jeweils optimalen Mengen an Herbizid und Antidot sind abhängig vom Typ des verwendeten Herbizids bzw. Antidots sowie von der Art des zu behandelnden Pflanzenbestandes und lassen sich von Fall zu Fall durch entsprechende Versuche ermitteln.

Als Haupteinsatzgebiete für die erfindungsgemäßen Mittel kommen vor allem Kulturen von Getreide (Weizen, Roggen, Gerste, Hafer), Reis, Mais und Hirse in Frage.

Die Erfindungsgemäßen Mittel können je nach ihren Eigenschaften zur Vorbehandlung des Saatgutes der Kulturpflanze (Beizung der Samen) oder vor der Saat in die Saatfurchen oder als Tankmischung vor ode rnach dem Auflaufen der Pflanzen verwendet werden. Vorauflaufbehandlung schließt sowohl die Behandlung der Anbaufläche vor der Aussaat als auch die Behandlung der angesäten, aber noch nicht bewachsenen Anbauflächen ein.

Grundsätzlich kann das Antidot vor, nach oder gleichzeitig mit einem Herbizid angewendet werden, bevorzugt ist jedoch die gleichzeitige Anwendung in Form von Tankmischungen oder gegebenenfalls Fertigformulierungen.

Die als Ausgangsprodukte für die Synthese benötigten Halogen-benzoxazole und Halogenbenzthiazole können nach literaturbekannten Methoden dargestellt werden [J. prakt. Chem. [2], *42*, 454; J. org. Chem. *23* (1958), 1500, 1502; DE—A—516 998; J. Ind. Chem. Soc. *10* (1933), 563, 569].

Für die notwendigen Aminosäuren und ihre Derivate sind in der Literatur ebenfalls zahlreiche Herstellungswege beschrieben [vgl. Houben-Weyl, Method. der org. Chem. Bd. XI/2 (1958), 269—511].

Beispiel 1

N-Methyl-N-[6-chlor-benzthiazolyl-(2)]-glycin

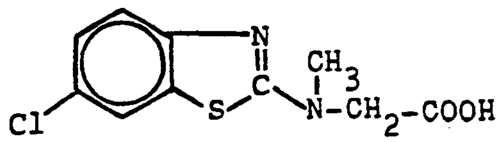

Im 1 Liter-Vierhalskolben mit Thermometer, Kühler mit $CaCl_2$-Trockenrohr und Gaseinleitungsrohr wurden 600 ml absol. Dimethyl-sulfoxid vorgelegt. Ein gelinder Strom trock. Stickstoffs wurde auf die Oberfläche der Flüssigkeit geleitet und unter Rühren rasch 66 g (1,65 Mol) NaOH (pulverisiert) und anschließend 73,5 g (0,825 Mol) N-Methyl-glycin (Sarkosin) in kleinen Portionen eingetragen. Unter gelinder Erwärmung bildet sich das Natrium-Salz des Sarkosins. — Es wurde 30 Min. bei 25—30°C nachgerührt, dann 153 g (0,75 Mol) 2,6-Dichlorbenzthiazol portionsweise in dem Maße zugegeben, daß die Temperatur der stark exothermen Reaktion nicht über 75—80°C anstieg. Danach wurde 5 Stunden bei 95—100°C gerührt, abgekühlt und das Gemisch in ca. 2 Liter Eiswasser eingegossen und angesäuert (pH 2—3). Das ausfallende Chlorbenzthiazolyl)-sarkosin wurde abgesaugt, mit kaltem Wasser säurefrei gewaschen und über $CaCl_2$ im Vakuum bei 50—60°C getrocknet. *Ausbeute*: 171 g (88,8% d.Th.), Fp.: 161—163°C.

Beispiel 2

N-Methyl-N-[6-chlor-benzthiazolyl-(2)]-glycin-ethylester

Der Ethylester der im Beispiel 1 hergestellten Aminosäure wurde durch 10-stündiges Erhitzen der freien Säure (145 g = 0,565 Mol) in 1100 ml absol. Äthanol unter Zuatz von 50 g konz. Schwefelsäure, anschließendes Abdestillieren der Hauptmenge des überschüssigen Alkohols und Eingießen der konz. Lösung in eiskalte wässerige $NaHCO_3$-Lösung erhalten. Der rohe Ester (151 g) wurde aus Ethanol umkristallisiert.

*Ausbeute*: 114 g (70,9% d.Th.), Fp.: 97—99°C.

Beispiel 3

N-[Benzoxazolyl-(2)]-glycin-ethylester

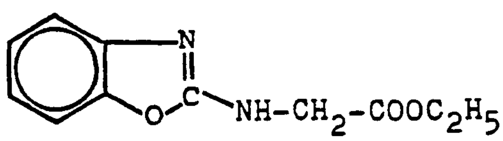

In ein Gemisch aus 23,0 g (0,15 Mol) 2-Chlorbenzoxazol, 250 ml absol. Acetonitril und 44,2 g (0,32 Mol) wasserfreiem $K_2CO_3$ wurden unter Rühren bei 50° im Laufe von 3 Stunden 22,3 g (0,16 Mol) Glycinethylester-hydrochlorid in kleinen Portionen eingerührt. Das Gemisch wurde noch 4 Stunden bei leichtem Rückfluß (80—82°C) nachgerührt, im Eiswasserbad abgekühlt und in ca. 750 ml Eiswasser eingegossen.

Das ausfallende Reaktionsprodukt wurde abgesaugt, mit Wasser und wenig eiskaltem Ethanol gewaschen und im Vakuum über $CaCl_2$ getrocknet. *Ausbeute*: 26,0 g (78,7% d.Th.), Fp.: 102—104°C.

In analoger Weise erhält man die folgenden Verbindungen.

**TABELLE 1:** Benzoxazolyl-(2)- und Benzthiazolyl-(2)-aminosäuren und Derivate

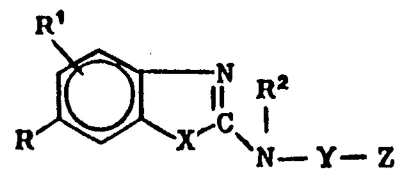

| Bsp. Nr. | R | $R^1$ | $R^2$ | X | Y | Z | Fp. (°C) |
|---|---|---|---|---|---|---|---|
| 4 | H | H | H | O | $-CH_2-$ | 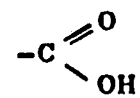 | 193-196 |
| 5 | H | $5-CH_3$ | H | O | $-CH_2-$ | 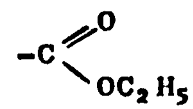 | 96-98 |
| 6 | H | $5-CH_3$ | $-CH_3$ | O | $-CH_2-$ | 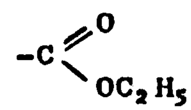 | 60-63 |
| 7 | Cl | H | H | O | $-CH_2-$ | 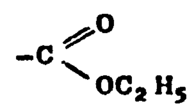 | 128-130 |
| 8 | Cl | H | H | O | $CH_3$<br>$-CH-$ | 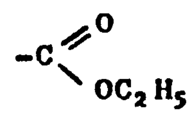 | 68 |
| 9 | H | H | $-CH_3$ | O | $-CH_2-$ | 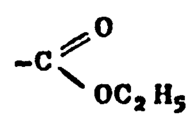 | 68-70 |

<u>Forts. Tabelle 1</u>

| Bsp. Nr. | R | $R^1$ | $R^2$ | X | Y | Z | Fp. [°C] |
|---|---|---|---|---|---|---|---|
| 10 | Cl | H | $-CH_3$ | O | $-CH_2-$ | 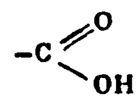 | 183–185 |
| 11 | Cl | H | $-CH_3$ | O | $-CH_2-$ | 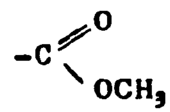 | 102–104 |
| 12 | Cl | H | $-CH_3$ | O | $-CH_2-$ | 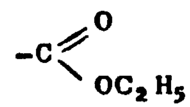 | 85–87 |
| 13 | Cl | H | $-CH_3$ | O | $-CH_2-$ | 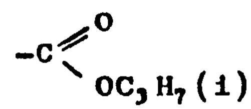 | 98–100 |
| 14 | Cl | H | $-CH_3$ | O | $-CH_2-$ | 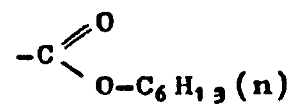 | 64–66 |
| 15 | Cl | H | $-CH_3$ | O | $-CH_2-$ | 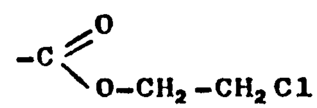 | 73–75 |
| 16 | Cl | H | $-CH_3$ | O | $-CH_2-$ | 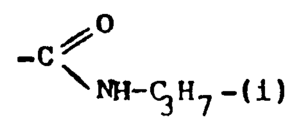 | 203–205 |

Forts. Tabelle 1

| Bsp. Nr. | R | $R^1$ | $R^2$ | X | Y | Z | Fp. [°C] |
|---|---|---|---|---|---|---|---|
| 17 | Cl | H | $-CH_3$ | O | $-CH_2-CH_2-$ | $-CN$ | 96-98 |
| 18 | H | H | H | O | $-CH_2-CH_2-CH_2-$ | 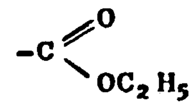 | 70-72 |
| 19 | Cl | H | H | O | 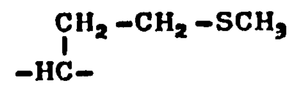 | 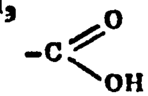 | 144-146 |
| 20 | Cl | H | H | O | 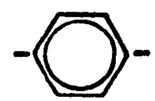 | 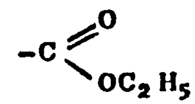 | 225-230 |
| 21 | H | H | H | O | $-CH_2-$ | 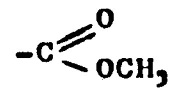 | 98 |
| 22 | H | H | $-CH_3$ | O | $-CH_2-$ | 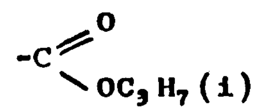 | 92 |
| 23 | H | H | $-CH_3$ | O | $-CH_2-$ | 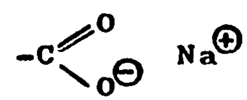 | |

<u>Forts. Tabelle 1</u>

| Bsp. Nr. | R | $R^1$ | $R^2$ | X | Y | Z | Fp. [°C] |
|---|---|---|---|---|---|---|---|
| 24 | Cl | H | $-CH_3$ | O | $-CH_2-$ | 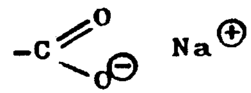 | |
| 25 | Cl | H | $-CH_3$ | O | $-CH_2-$ | 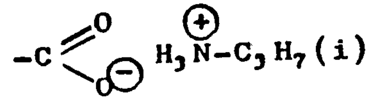 | |
| 26 | H | H | H | S | $-CH_2-$ | 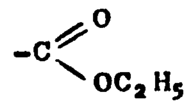 | 82-83 |
| 27 | H | H | H | S | $CH_3$<br>$-CH-$ | 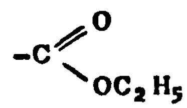 | 63-65 |
| 28 | H | H | H | S | $-CH_2-CH_2-$ | 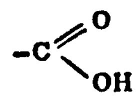 | 200-203 |
| 29 | H | H | H | S | $-CH_2-CH_2-$ | 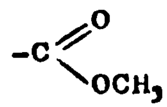 | 91-94 |
| 30 | H | H | H | S | $-CH_2-CH_2-$ | 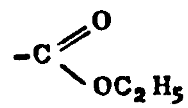 | 87-88 |
| 31 | H | H | H | S | $-CH_2-CH_2-CH_2-$ | 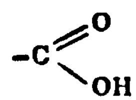 | 132-134 |
| 32 | H | H | H | S | $-CH_2-CH_2-CH_2-$ | 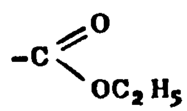 | 65-66 |

**Forts. Tabelle 1**

| Bsp. Nr. | R | $R^1$ | $R^2$ | X | Y | Z | Fp. [°C] |
|---|---|---|---|---|---|---|---|
| 33 | Cl | H | H | S | $-CH_2-$ | 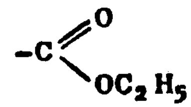 | 144-146 |
| 34 | Cl | H | H | S | $CH_3$<br>$-CH-$ | 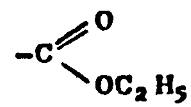 | 111-113 |
| 35 | Cl | H | H | S | $-CH_2-CH_2-$ | 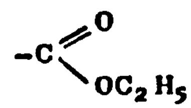 | 110-112 |
| 36 | Cl | H | H | S | $-CH_2-CH_2-$ | 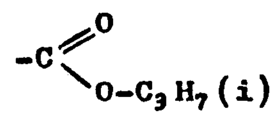 | 115-117 |
| 37 | Cl | H | H | S | $C_2H_5$<br>$-CH-$ | 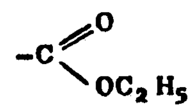 | 105-107 |
| 38 | Cl | H | H | S | $-CH_2-CH_2-CH_2-$ | 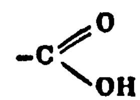 | 178-179 |
| 39 | Cl | H | H | S | $-CH_2-CH_2-CH_2-$ | 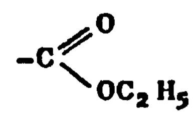 | 120-121 |

Forts. Tabelle 1

| Bsp. Nr. | R | $R^1$ | $R^2$ | X | Y | Z | Fp. [°C] |
|---|---|---|---|---|---|---|---|
| 40 | Cl | H | H | S | 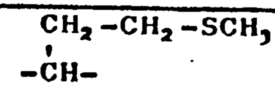 | 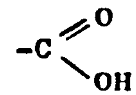 | 159-162 |
| 41 | Cl | H | H | S | 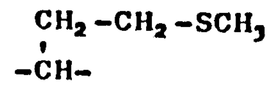 | 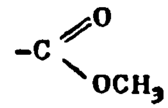 | 79-83 |
| 42 | Cl | H | H | S | 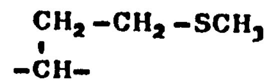 | 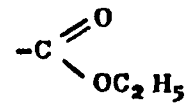 | 80-82 |
| 43 | Cl | H | H | S | 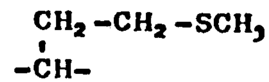 | 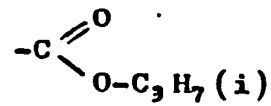 | 91-92 |
| 44 | Cl | H | H | S | 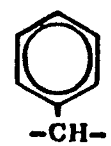 | 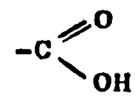 | 231-35 (z) |
| 45 | Cl | H | H | S | 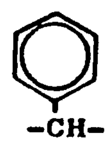 | 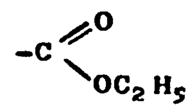 | 134-138 |
| 46 | Cl | H | H | S | 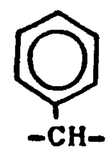 | 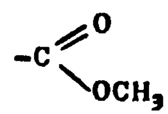 | 179-182 |

**Forts. Tabelle 1**

| Bsp. Nr. | R | $R^1$ | $R^2$ | X | Y | Z | Fp. [°C] |
|---|---|---|---|---|---|---|---|
| 47 | Cl | H | H | S | 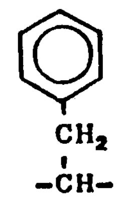 | 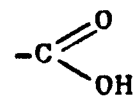 | |
| 48 | Cl | H | H | S | 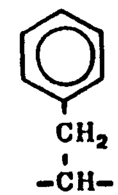 | 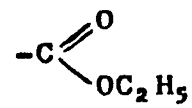 | |
| 49 | Cl | H | H | S | 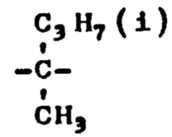 | 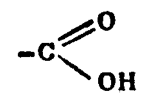 | |
| 50 | Cl | H | H | S | 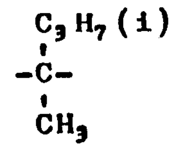 | 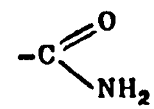 | |

Forts. Tabelle 1

| Bsp. Nr. | R | $R^1$ | $R^2$ | X | Y | Z | Fp. [°C] |
|---|---|---|---|---|---|---|---|
| 51 | Cl | H | H | S | 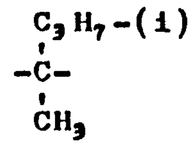 | 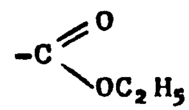 | |
| 52 | Cl | H | H | S | 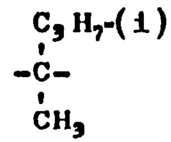 | -CN | |
| 53 | Cl | H | $-CH_3$ | S | $-CH_2-$ | 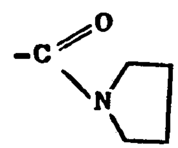 | |
| 54 | Cl | H | $-CH_3$ | S | $-CH_2-$ | 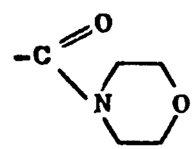 | 150-152 |

**Forts. Tabelle 1**

| Bsp. Nr. | R | $R^1$ | $R^2$ | X | Y | Z | Fp. [°C] |
|---|---|---|---|---|---|---|---|
| 55 | H | H | $-CH_3$ | S | $-CH_2-$ | 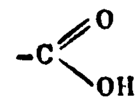 | 160-162 |
| 56 | H | H | $-CH_3$ | S | $-CH_2-$ | 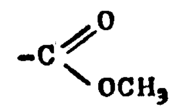 | 91-93 |
| 57 | H | H | $-CH_3$ | S | $-CH_2-$ | 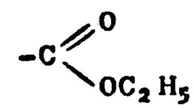 | 41-43 |
| 58 | H | H | $-CH_3$ | S | $-CH_2-$ | 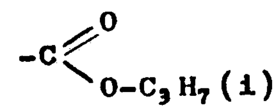 | 47-49 |
| 59 | H | H | $-CH_3$ | S | $-CH_2-$ | 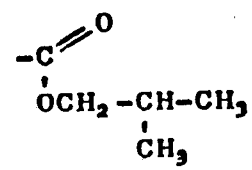 | 56-58 |
| 60 | H | H | $-CH_3$ | S | $-CH_2-$ | 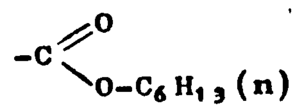 | 47-49 |
| 61 | H | H | $-CH_3$ | S | $-CH_2-$ | 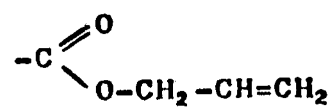 | 55-57 |

**Forts. Tabelle 1**

| Bsp. Nr. | R | $R^1$ | $R^2$ | X | Y | Z | Fp. [°C] |
|---|---|---|---|---|---|---|---|
| 62 | H | H | $-CH_3$ | S | $-CH_2-$ | 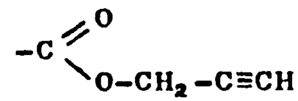 | 99-103 |
| 63 | H | H | $-CH_3$ | S | $-CH_2-CH_2-$ | -CN | 90-92 |
| 64 | Cl | H | $-CH_3$ | S | $-CH_2-$ | 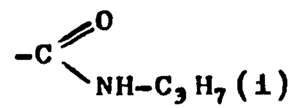 | 200-203 |
| 65 | Cl | H | $-CH_3$ | S | $-CH_2-$ | 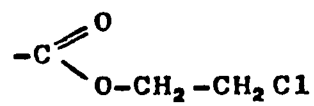 | 95-98 |
| 66 | Cl | H | $-CH_3$ | S | $-CH_2-CH_2-$ | -CN | 115-117 |
| 67 | Cl | 4-Cl | $-CH_3$ | S | $-CH_2-$ | 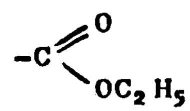 | 143-144 |
| 68 | Cl | 4-Cl | $-CH_3$ | S | $-CH_2-$ | 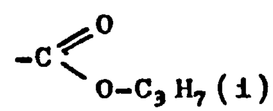 | 121-122 |
| 69 | H | H | $-CH_3$ | S | $-CH_2-$ | 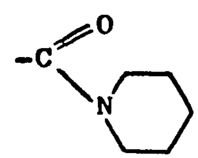 | 119-121 |

**Forts. Tabelle 1**

| Bsp. Nr. | R | $R^1$ | $R^2$ | X | Y | Z | Fp. [°C] |
|---|---|---|---|---|---|---|---|
| 70 | H | H | $-CH_3$ | S | $-CH_2-$ | 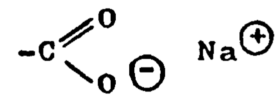 | 284–285 (Z) |
| 71 | H | H | $-CH_3$ | S | $-CH_2-$ | 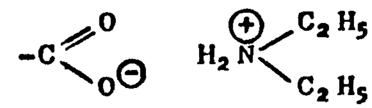 | 118–120 |
| 72 | Cl | H | $-CH_3$ | S | $-CH_2-$ | 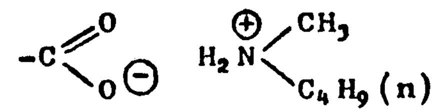 | 141–143 |
| 73 | Cl | H | $-CH_3$ | S | $-CH_2-$ | 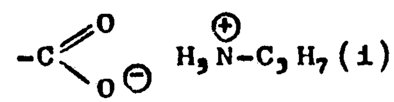 | 195–197 |
| 74 | Cl | H | $-CH_3$ | S | $-CH_2-CH_2-$ | 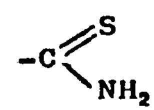 | 184 –186 |
| 75 | H | H | H | O | $-CH_2-$ | $-COOCH(CH_3)_2$ | 115 |
| 76 | Cl | H | $-CH_3$ | S | $-CH_2-$ | $-COOC_8H_{17}$(n) | 63-64 |

FORMULIERUNGSBEISPIELE:

Beispiel A

Ein emulgierbares Konzentrat wird erhalten aus
15 Gewichtsteilen Wirkstoff
75 Gewichtsteilen Cyclohexanon als Lösungsmittel
10 Gewichtsteilen oxäthyliertes Nonylphenol (10 AeO) als Emulgator

Beispiel B

Ein Stäubemittel wird erhalten, indem man
10 Gewichtsteile Wirkstoff
90 Gewichtsteile Talkum als Inertstoff
mischt und in einer Schlagmühle zerkleinert.

Beispiel C

Ein in Wasser leicht dispergierbares, benetzbares Pulver wird erhalten, indem man
25 Gewichtsteile Wirkstoff
64 Gewichtsteile kaolinhaltiges Quarz als Inertstoff
10 Gewichtsteile ligninsulfonsaures Kalium und
1 Gewichtsteil oleylmethyltaurinsaures Natrium als Netz- und Dispergiermittel
mischt und in einer Stiftmühle mahlt.

BIOLOGISCHE BEISPIELE

Beispiel 1

Weizen und Mais wurden im Gewächshaus bis zum 5-Blattstadium herangezogen und dann mit einem Herbizid und erfindungsgemäßen Mitteln bei einer Sprühwasseraufwandmenge von 600 l/ha behandelt. 3 Wochen nach der Behandlung wurden die Pflanzen auf jede Art von Schädigung bonitiert, wobei insbesondere das Ausmaß der anhaltenden Wachstumshemmung betrachtet wurde.

Die Ergebnisse aus Tabelle 1 veranschaulichen, daß die erfindungsgemäßen Verbindungen starke Herbizidschäden sehr effektiv reduzieren können.

Mischungen aus den verwendeten Herbiziden und erfindungsgemäßen Mitteln eignen sich also zur selektiven Unkrautbekämpfung in Getreide und Mais.

Die Wirksamkeit der verschiedenen Herbizide wurde durch die Zugabe der erfindungsgemäßen Safener nicht beeinträchtigt; sie betrug bei den angegebenen Aufwandmengen von 0.25 bzw. 1.5 kg AS durchweg 100% z.B. bei Ackerfuchsschwanz (Alopecurus myosuroides), bei Flughafer (Avena fatua) und bei Hirsen wie Echinochloa- und Setaria-Arten.

Die erfindungsgemäßen Mittel allein verursachten in den Kulturpflanzen bei den angewendeten Konzentrationen (2.5 kg/ha) keinerlei Schäden (durchweg 0%).

TABELLE 1

Antidot-Wirkung bei Blattapplikation mit verschiedenen Herbiziden (Schädigung der Kulturpflanzen in %)

| Verbindung (Beispiel) | Dosis (kg/ha) | Schädigung bei ZM | TA |
|---|---|---|---|
| H1 | 0.25 | 80 | — |
| H2 | 1.5 | — | 80 |
| | 2.0 | — | 85 |
| H3 | 0.4 | 95 | — |
| H1 + 2 | 0.25 + 2.5 | 15 | — |
| H1 + 26 | 0.25 + 2.5 | 20 | — |
| H1 + 57 | 0.25 + 2.5 | 25 | — |
| H2 + 2 | 1.5 + 2.5 | — | 20 |
| H2 + 9 | 2.0 + 2.5 | — | 40 |
| H2 + 21 | 2.0 + 2.5 | — | 40 |
| H2 + 56 | 2.0 + 2.5 | — | 40 |
| H2 + 57 | 1.5 + 2.5 | — | 10 |
| H2 + 58 | 2.0 + 2.5 | — | 25 |
| H2 + 59 | 2.0 + 2.5 | — | 35 |
| H3 + 3 | 0.4 + 2.5 | 50 | — |
| H3 + 21 | 0.4 + 2.5 | 35 | — |
| H3 + 61 | 0.4 + 2.5 | 60 | — |
| H3 + 62 | 0.4 + 2.5 | 60 | — |

Abkürzungen:
ZM = Zea mays (Mais), TA = Triticum aestivum (Weizen)
H1 = 2-[4-(4-Brom-2-chlor-phenoxy)phenoxy]-propionsäuremethylester (DE—A—26 01 548)
H2 = 2-[4-(6-Chlor-benzoxazol-2-yl-oxy)-phenoxy]-propionsäure-ethylester; Fenoxaprop-ethyl (DE—A—26 40 730)
H3 = 2-[4-(2,4-Dichlorphenoxy)-phenoxy]-propionsäuremethylester; Dichlofop-methyl (DE—A—22 23 894).

**Patentansprüche für die Vertragsstaaten: BH CH DE FR GB IT LI NL SE**

1. Verwendung von Verbindungen der Formel I

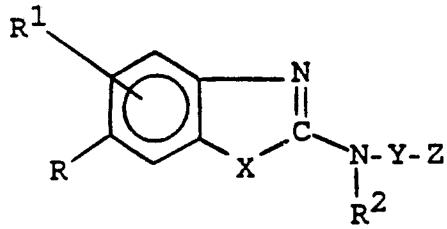

(I)

worin

X = O oder S;

R, $R^1$ = Wasserstoff, Halogen, $(C_1—C_4)$-Alkyl, $(C_1—C_4)$-Alkoxy, CN, $NO_2$ oder $CF_3$;

$R^2$ = Wasserstoff oder $(C_1—C_4)$-Alkyl;

Y = geradkettiges oder verzweigtes $(C_1—C_8)$-Alkylen, von dem sich 1—3 C-Atome in gerader Kette zwischen dem Amin-Stickstoff und der Gruppe Z befindet; Methylthio-$(C_1—C_3)$-alkylen, Phenyl-$(C_1—C_3)$-alkylen oder Phenylen;

Z = 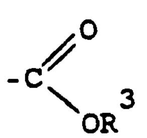 , 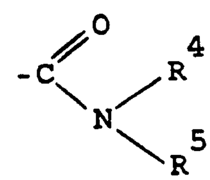 , -CN oder 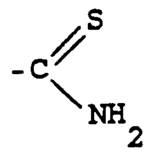 ;

$R^3$ = H, $(C_1—C_8)$-Alkyl, das ggf. durch Halogen oder $(C_1—C_2)$-Alkoxy substituiert sein kann, $(C_3—C_4)$-Alkenyl, $(C_3—C_4)$-Alkinyl oder das Kation einer anorganischen oder organischen Base;

$R^4$ = H, $(C_1—C_6)$-Alkyl;

$R^5$ = H, $(C_1—C_6)$-Alkyl oder Phenyl, das ggf. 1- oder 2-fach durch Halogen, $(C_1—C_4)$-Alkyl oder $(C_1—C_4)$-Alkoxy substituiert sein kann;

$R^4$ und $R^5$ zusammen mit dem Stickstoffatom einen Pyrrolidino-, Piperidino- oder Morpholino-Rest bedeuten,

zum Schutz von Nutzpflanzen gegen phytotoxische Wirkungen von Herbiziden.

2. Verwendung gemäß Anspruch 1, worin R, $R^1$, $R^2$, $R^3$, $R^4$, $R^5$, X, Y und Z die angegebenen Bedeutungen haben, mit der Maßgabe, daß mindestens einer der Reste R, $R^1$ und $R^2$ von Wasserstoff verschieden ist.

3. Verwendung gemäß Anspruch 1, dadurch gekennzeichnet, daß in den Verbindungen der Formel I

Y O oder S

R H, $CH_3$ oder Cl

$R^1$ H

$R^2$ H oder $CH_3$

Y $C_1$- oder $C_2$-Alkylen,

Z einen Rest der Formel —$COOR^3$ und $R^3$ $(C_1—C_8)$Alkyl, $(C_3—C_4)$Alkenyl, $(C_3—C_4)$-Alkinyl oder ein Kation bedeuten.

4. Verbindungen der Formel (I) nach Anspruch 3, worin R, $R^1$, $R^2$, $R^3$, $R^4$, $R^5$, X, Y und Z die angegebenen Bedeutungen haben, mit der Maßgabe, daß mindestens einer der Reste R und $R^2$ von Wasserstoff verschieden ist.

5. N-Methyl-N-(6-chlor-benzthiazolyl-(2))-glycinethylester.

6. N-Methyl-N-benzoazolyl-(2)-glycinethylester.

7. N-Methyl-N-benzthiazolyl-(2)-glycinethylester.

8. N-Methyl-N-benzthiazolyl-(2)-glycinisobutylester.

9. Verfahren zur Herstellung von Verbindungen der Formel I gemäß Anspruch 4, dadurch gekennzeichnet, daß man

a) Verbindungen der Formel

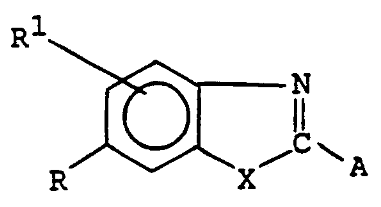

(II)

worin A eine leicht abspaltbare Gruppe ("leaving group") bedeutet, mit Verbindungen der Formel

$$\underset{\displaystyle R^2}{\underset{|}{HN}}—Y—COOR^3 \qquad (III)$$

oder

b) Verbindungen der Formel

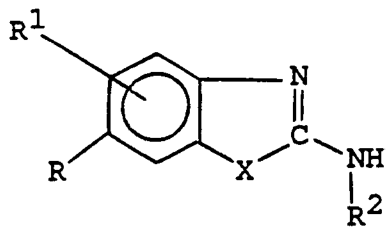

(IV)

mit Verbindungen der Formel

Hal—Y—$COOR^3$ (V)

umsetzt und gewünschtenfalls die erhaltenen Verbindungen in an sich bekannter Weise, z.B. durch Veresterung, Verseifung, Salzbildung, Amidierung, Alkylierung oder Dehydratisierung in andere Verbindungen der Formel I überführt.

10. Pflanzenschützende Kombination, gekennzeichnet durch einen Gehalt an

a) einem Herbizid aus der Reihe der Phenoxyphenoxy-, Benzoxazolyloxyphenoxy-, Benzthiazolyloxyphenoxy- oder Benzylphenoxy-carbonsäureester und

b) einer Verbindung der Formel I gemäß Anspruch 1.

11. Pflanzenschutzende Kombination gemäß Anspruch 10, dadurch gekennzeichnet, daß das Herbizid (a) Diclofop-methyl oder Fenoxaprop-ethyl ist.

12. Verfahren zum Schützen von Nutzpflanzen gegen phytotoxische Nebenwirkungen von Herbiziden, dadurch gekennzeichnet, daß man die zu schützenden Pflanzen bzw. die Anbaufläche vor, nach oder gleichzeitig mit der Anwendung eines Herbizides aus der Reihe der Phenoxyphenoxy-, Benzoxazolyloxyphenoxy-, Benzthiazolyloxyphenoxy- oder Benzylphenoxycarbonsäureester mit einer Verbindung der Formel I gemäß Anspruch 1 behandelt.

**Patentansprüche für den Vertragsstaat: AT**

1. Verwendung von Verbindungen der Formel I

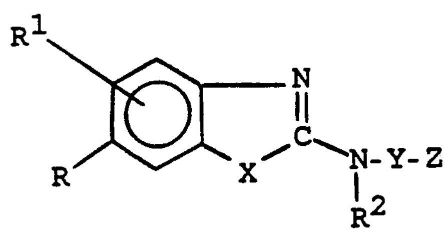

(I)

worin

X = O oder S;

R, $R^1$ = Wasserstoff, Halogen, ($C_1$—$C_4$)-Alkyl, ($C_1$—$C_4$)-Alkoxy, CN, $NO_2$ oder $CF_3$;

$R^2$ = Wasserstoff oder ($C_1$—$C_4$)-Alkyl;

Y = geradkettiges oder verzweigtes ($C_1$—$C_8$)-Alkylen, von dem sich 1—3 C-Atome in gerader Kette zwischen dem Amin-Stickstoff und der Gruppe Z befindet; Methylthio-($C_1$—$C_3$)-alkylen, Phenyl-($C_1$—$C_3$)-alkylen oder Phenylen;

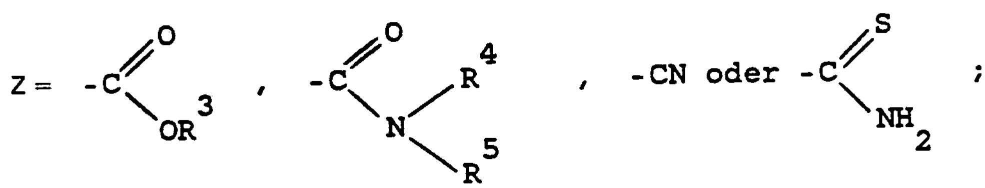

$R^3$ = H, ($C_1$—$C_8$)-Alkyl, das ggf. durch Halogen oder ($C_1$—$C_2$)-Alkoxy substituiert sein kann, ($C_3$—$C_4$)-Alkenyl, ($C_3$—$C_4$)-Alkinyl oder das Kation einer anorganischen oder organischen Base;

$R^4$ = H, ($C_1$—$C_6$)-Alkyl;

$R^5$ = H, ($C_1$—$C_6$)-Alkyl oder Phenyl, das ggf. 1- oder 2-fach durch Halogen, ($C_1$—$C_4$)-Alkyl oder ($C_1$—$C_4$)-Alkoxy substituiert sein kann;

$R^4$ und $R^5$ zusammen mit dem Stickstoffatom einen Pyrrolidino-, Piperidino- oder Morpholino-Rest bedeuten,

zum Schutz von Nutzpflanzen gegen phytotoxische Wirkungen von Herbiziden.

2. Verwendung gemäß Anspruch 1, worin R, $R^1$, $R^2$, $R^3$, $R^4$, $R^5$, X, Y und Z die angegebenen

Bedeutungen haben, mit der Maßgabe, daß mindestens einer der Reste R, $R^1$ und $R^2$ von Wasserstoff verschieden ist.

3. Verwendung gemäß Anspruch 1, dadurch gekennzeichnet, daß in den Verbindungen der Formel I
Y O oder S
R H, $CH_3$ oder Cl
$R^1$ H
$R^2$ H oder $CH_3$
Y $C_1$- oder $C_2$-Alkylen,
Z einen Rest der Formel —$COOR^3$ und $R^3$ ($C_1$—$C_8$)Alkyl, ($C_3$—$C_4$)Alkenyl, ($C_3$—$C_4$)-Alkinyl oder ein Kation bedeuten.

4. Verwendung nach Anspruch 3, worin R, $R^1$, $R^2$, $R^3$, $R^4$, $R^5$, X, Y und Z die angegebenen Bedeutungen haben, mit der Maßgabe, daß mindestens einer der Reste R und $R^2$ von Wasserstoff verschieden ist.

5. Verwendung nach Anspruch 3, dadurch gekennzeichnet, daß die Verbindung der Formel (I) N-Methyl-N-(6-chlor-benzthiazolyl-(2))-glycinethylester verwendet wird.

6. Verwendung nach Anspruch 3, dadurch gekennzeichnet, daß die Verbindung der Formel (I) N-Methyl-N-benzoazolyl-(2)-glycinethylester ist.

7. Verwendung nach Anspruch 3, dadurch gekennzeichnet, daß die Verbindung der Formel (I) N-Methyl-N-benzthiazolyl-(2)-glycinethylester ist.

8. Verwendung nach Anspruch 3, dadurch gekennzeichnet, daß die Verbindung der Formel (I) N-Methyl-N-benzthiazolyl-(2)-glycinisobutylester ist.

9. Verfahren zur Herstellung von Verbindungen der nach Anspruch 4, definierten Formel I, dadurch gekennzeichnet, daß man

a) Verbindungen der Formel

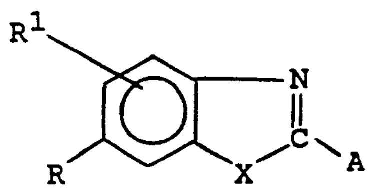

(II)

worin A eine leicht abspaltbare Gruppe ("leaving group") bedeutet, mit Verbindungen der Formel

$$HN(R^2)—Y—COOR^3 \qquad (III)$$

oder

b) Verbindungen der Formel

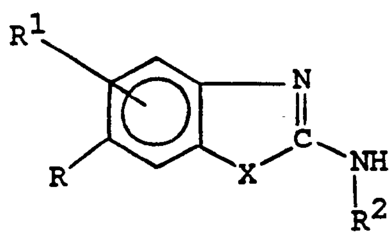

(IV)

mit Verbindungen der Formel

$$Hal—Y—COOR^3 \qquad (V)$$

umsetzt und gewünschtenfalls die erhaltenen Verbindungen in an sich bekannter Weise, z.B. durch Veresterung, Verseifung, Salzbildung, Amidierung, Alkylierung oder Dehydratisierung in andere Verbindungen der Formel I überführt.

10. Pflanzenschützende Kombination, gekennzeichnet durch einen Gehalt an

a) einem Herbizid aus der Reihe der Phenoxyphenoxy-, Benzoxazolyloxyphenoxy-, Benzthiazolyloxyphenoxy- oder Benzylphenoxy-carbonsäureester und

b) einer Verbindung der Formel I gemäß Anspruch 1.

11. Pflanzenschützende Kombination gemäß Anspruch 10, dadurch gekennzeichnet, daß das Herbizid (a) Diclofop-methyl oder Fenoxaprop-ethyl ist.

12. Verfahren zum Schützen von Nutzpflanzen gen phytotoxische Nebenwirkungen von Herbiziden, dadurch gekennzeichnet, daß man die zu schützenden Pflanzen bzw. die Anbaufläche vor, nach oder gleichzeitig mit der Anwendung eines Herbizides aus der Reihe der Phenoxyphenoxy-,

Benzoxazolyloxyphenoxy-, Benzthiazolyloxyphenoxy- oder Benzylphenoxycarbonsäure-ester mit einer Verbindung der Formel I gemäß Anspruch 1 behandelt.

**Revendications pour les Etats contractants: BE CH DE FR GB IT LI NL SE**

1. L'emploi, pour protéger des plantes utiles contre des effets phytotoxiques d'herbicides, des composés de formule I:

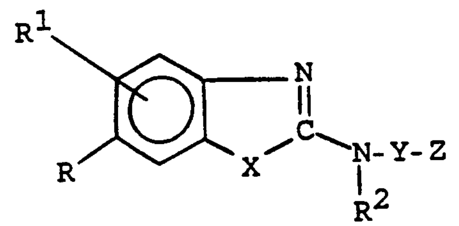

(I)

dans laquelle:

X représente l'oxygène ou le soufre;

$R^1$ et $R^2$ représentent chacun l'hydrogène, un halogène, un alkyle ou un alcoxy en $C_1$—$C_4$ ou le groupe CN, $NO_2$ ou $CF_3$;

$R^2$ désigne l'hydrogène ou un alkyle en $C_1$—$C_4$;

Y un alkylène à chaîne linéaire ou ramifié en $C_1$—$C_8$ dont de 1 à 3 atomes de carbone se trouvent en chaîne linéaire entre l'azote et le groupe Z; ou un groupe méthylthio-($C_1$—$C_3$)-alkylène, phényl-($C_1$—$C_3$)-alkylène ou phénylène; et

Z désigne un groupe

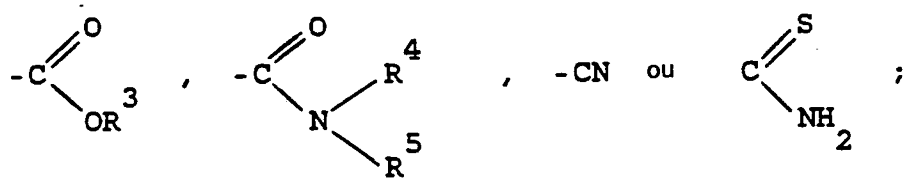

$R^3$ étant l'hydrogène, un alkyle en $C_1$—$C_8$ pouvant éventuellement porter un halogène ou un alcoxy en $C_1$ ou $C_2$, un alcényle ou un alcynyle en $C_3$—$C_4$ ou le cation d'une base minérale ou organique,

$R^4$ étant l'hydrogène ou un alkyle en $C_1$—$C_6$; et

$R^5$ l'hydrogène, un alkyle en $C_1$—$C_6$ ou un phényle portant éventuellement un ou deux atomes d'halogènes ou alkyles ou alcoxy en $C_1$—$C_4$; ou bien

$R^4$ et $R^5$ forment ensemble et avec l'atome d'azote un radical pyrrolidino, pipéridino ou morpholino.

2. Emploi selon la revendication 1 dans lequel, dans la formule indiquée, parmi R, $R^1$ et $R^2$ l'un au moins n'est pas l'hydrogène.

3. Emploi selon la revendication 1 caractérisé en ce que dans les composés de formule I:

Y est l'hydrogène ou le soufre,

R l'hydrogène, le groupe méthyle ou le chlore,

$R^1$ l'hydrogène,

$R^2$ l'hydrogène ou le groupe méthyle,

Y un alkylène en $C_1$ ou $C_2$, et

Z un radical —$COOR^3$, $R^3$ désignant un alkyle en $C_1$—$C_8$, un alcényle ou un alcynyle en $C_3$—$C_4$ ou un cation.

4. Les composés de formule I selon la revendication 3 dans lesquels R, $R^1$, $R^2$, $R^3$, $R^4$, $R^5$, X, Y et Z ont les significations indiquées, mais parmi R et $R^2$ l'un des deux au moins n'est pas l'hydrogène.

5. Ester éthylique de N-méthyl-N-(6-chlorobenzothiazolyl-(2))-glycine.

6. Ester éthylique de N-méthyl-N-benzoxazolyl-(2)-glycine.

7. Ester éthylique de N-méthyl-N-benzothiazolyl-(2)-glycine.

8. Ester isobutylique de N-méthyl-N-benzothiazolyl-(2)-glycine.

9. Procédé de préparation des composés de formule I de la revendication 4, procédé caractérisé en ce que l'on fait réagir:

a) des composés de formule:

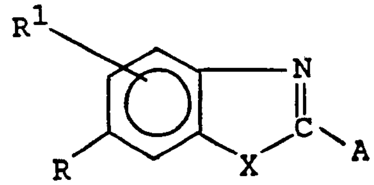

(II)

A désignant un groupe facilement éliminable, avec des composés de formule:

$$HN-Y-COOR^3$$
$$|$$
$$R^2$$

(III)

ou bien

b) des composés de formule:

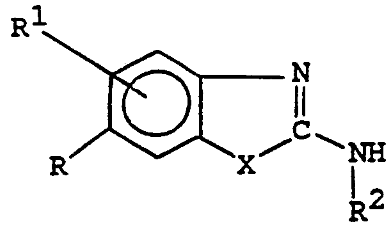

(IV)

avec des composés de formule:

$$Hal-Y-COOR^3 \qquad (V)$$

et le cas échéant, si l'on veut, on transforme les composés ainsi obtenus, de manière connue, en d'autres composés de formule I, par exemple par estérification, saponification, salification, amidation, alkylation ou déshydratation.

10. Association de produits pour la protection de plantes, caractérisée en ce qu'elle comprend:

a) un herbicide de la série des esters d'acides phénoxyphénoxy-, benzoxazolyloxyphénoxy-, benzothiazolyloxyphénoxy- et benzylphénoxy-carboxyliques, avec

b) un composé de formule I selon la revendication 1.

11. Association protectrice de plantes selon la revendication 10, caractérisée en ce que l'herbicide (a) est le dichlofop-méthyl ou le fénoxaprop-éthyl.

12. Procédé pour protéger des plantes utiles contre les effets secondaires phytotoxiques d'herbicides, procédé caractérisé en ce que l'on traite les plantes à protéger ou les surfaces de culture de ces plantes, avant, après ou en même temps qu'avec un herbicide de la série des esters d'acides phénoxyphénoxy-, benzoxazolyloxyphénoxy-, benzothiazolyloxyphénoxy- et benzylphénoxy-carboxyliques, avec un composé de formule I de la revendication 1.

**Revendications pour l'Etat contractants: AT**

1. L'Emploi, pour protéger des plantes utiles contre des effets phytotoxiques d'herbicides, des composés de formule I:

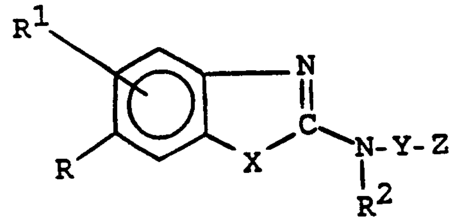

(I)

dans laquelle:

X représente l'oxygène ou le soufre;

$R^1$ et $R^2$ représentent chacun l'hydrogène, un halogène, un alkyle ou un alcoxy en $C_1$—$C_4$ ou le groupe CN, $NO_2$ ou $CF_3$;

$R^2$ désigne l'hydrogène ou un alkyle en $C_1$—$C_4$;

Y un alkylène à chaîne linéaire ou ramifié en $C_1$—$C_8$ dont de 1 à 3 atomes de carbone se trouvent en chaîne linéaire entre l'azote et le groupe Z; ou un groupe méthylthio-($C_1$—$C_3$)-alkylène, phényl-($C_1$—$C_3$)-alkylène ou phénylène; et

Z désigne un groupe

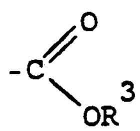 , 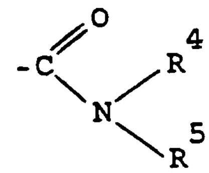 , -CN ou 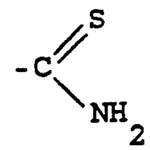 ;

$R^3$ étant l'hydrogène, un alkyle en $C_1$—$C_8$ pouvant éventuellement porter un halogène ou un alcoxy en $C_1$ ou $C_2$, un alcényle ou un alcynyle en $C_3$—$C_4$ ou le cation d'une base minérale ou organique,

$R^4$ étant l'hydrogène ou un alkyle en $C_1$—$C_6$; et

$R^5$ l'hydrogène, un alkyle en $C_1$—$C_6$ ou un phényle portant éventuellement un ou deux atomes d'halogènes ou alkyles ou alcoxy en $C_1$—$C_4$; ou bien

$R^4$ et $R^5$ forment ensemble et avec l'atome d'azote un radical pyrrolidino, pipéridino ou morpholino.

2. Emploi selon la revendication 1 dans lequel, dans la formule indiquée, parmi R, $R^1$ et $R^2$ l'un au moins n'est pas l'hydrogène.

3. Emploi selon la revendication 1 caractérisé en ce que dans les composés de formule I:

Y est l'hydrogène ou le soufre,

R l'hydrogène, le groupe méthyle ou le chlore,

$R^1$ l'hydrogène,

$R^2$ l'hydrogène ou le groupe méthyle,

Y un alkylène en $C_1$ ou $C_2$, et

Z un radical —$COOR^3$, $R^3$ désignant un alkyle en $C_1$—$C_8$, un alcényle ou un alcynyle en $C_3$—$C_4$ ou un cation.

4. Emploi selon la revendication 3 dans lequel, dans les formules indiquées, R, $R^1$, $R^2$, $R^3$, $R^4$, $R^5$, X, Y et Z ont les significations indiquées, mais parmi R et $R^2$ l'un des deux au moins n'est pas l'hydrogène.

5. Emploi selon la revendication 3, caractérisé en ce que le composé de formule I employé est l'ester éthylique de N-méthyl-N-(6-chloro-benzothiazolyl-(2))-glycine.

6. Emploi selon la revendication 3, caractérisé en ce que le composé de formule I employé est l'ester éthylique de N-méthyl-N-benzoxazolyl-(2)-glycine.

7. Emploi selon la revendication 3, caractérisé en ce que le composé de formule I employé est l'ester éthylique de N-méthyl-N-benzothiazolyl-(2)-glycine.

8. Emploi selon la revendication 3, caractérisé en ce que le composé de formule I employé est l'ester isobutylique de N-méthyl-N-benzothiazolyl-(2)-glycine.

9. Procédé de préparation des composés employés à la revendication 4, de formule I définie, procédé caractérisé en ce que l'on fait réagir:

a) des composés de formule:

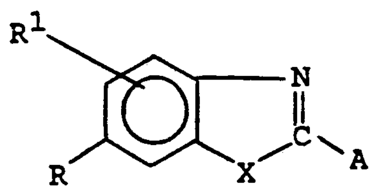

(II)

A désignant un groupe facilement éliminable, avec des composés de formule:

$$\underset{\displaystyle R^2}{\underset{|}{HN}}—Y—COOR^3 \qquad \text{(III)}$$

ou bien

b) des composés de formule:

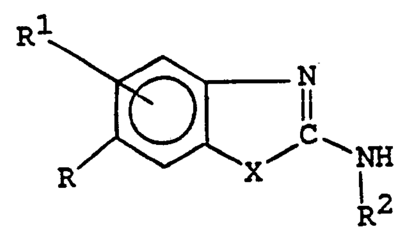

(IV)

avec des composés de formule:

$$Hal—Y—COOR^3 \qquad (V)$$

et le cas échéant, si l'on veut, on transforme les composés ainsi obtenus, de manière connue, en d'autres composés de formule I, par exemple par estérification, saponification, salification, amidation, alkylation ou déshydratation.

10. Association de produits pour la protection de plantes, caractérisée en ce qu'elle comprend:

a) un herbicide de la série des esters d'acides phénoxyphénoxy-, benzoxazolyloxyphénoxy-, benzothiazolyloxyphénoxy- et benzylphénoxy-carboxyliques, avec

b) un composé de formule I selon la revendication 1.

11. Association protectrice de plantes selon la revendication 10, caractérisée en ce que l'herbicide (a) est le dichlofop-méthyl ou le fénoxaprop-éthyl.

12. Procédé pour protéger des plantes utiles contre les effets secondaires phytotoxiques d'herbicides, procédé caractérisé en ce que l'on traite les plantes à protéger ou les surfaces de culture de ces plantes, avant, après ou en même temps qu'avec un herbicide de la série des esters d'acides phénoxyphénoxy-, benzoxazolyloxyphénoxy-, benzothiazolyloxyphénoxy- et benzylphénoxy-carboxyliques, avec un composé de formule I de la revendication 1.

**Claims for the Contracting States: BE CH DE FR GB IT LI NL SE**

1. The use of a compound of the formula I

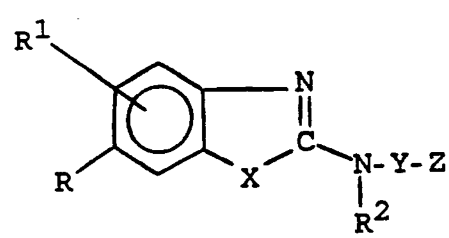

(I)

in which

X denotes O or S;

R and $R^1$ denote hydrogen, halogen, $(C_1—C_4)$-alkyl, $(C_1—C_4)$-alkoxy, CN, $NO_2$ or $CF_3$;

$R^2$ denotes hydrogen or $(C_1—C_4)$-alkyl;

Y denotes straight-chain or branched $(C_1—C_8)$-alkylene, 1—3 carbon atoms of which are in a straight chain between the amine nitrogen and the group Z; or denotes methylthio-$(C_1—C_3)$-alkylene, phenyl-$(C_1—C_3)$-alkylene or phenylene;

Z denotes

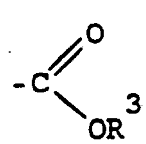 , 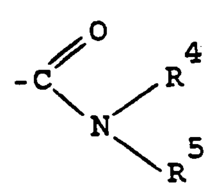 , -CN or 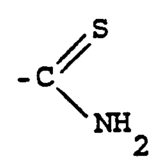 ;

$R^3$ denotes H, $(C_1—C_8)$-alkyl, which can optionally be substituted by halogen or $(C_1—C_2)$-alkoxy, $(C_3—C_4)$-alkenyl, $(C_3—C_4)$-alkynyl or the cation of an inorganic or organic base;

$R^4$ denotes H or $(C_1—C_6)$-alkyl;

$R^5$ denotes H, $(C_1—C_6)$-alkyl or phenyl, which can optionally be mono- or disubstituted by halogen, $(C_1—C_4)$-alkyl or $(C_1—C_4)$-alkoxy; or

$R^4$ and $R^5$ together with the nitrogen atom denote a pyrrolidino, piperidino or morpholino radical for protecting useful plants from the phytotoxic effects of herbicides.

2. The use as claimed in claim 1, wherein R, $R^1$, $R^2$, $R^3$, $R^4$, $R^5$, X, Y and Z have the meanings given, with the proviso that at least one of the radicals R and $R^2$ is other than hydrogen.

3. The use as claimed in claim 1, wherein, in a compound of the formula I,

X denotes O or S,

R denotes H, $CH_3$ or Cl,

$R^1$ denotes H,

$R^2$ denotes H or $CH_3$,

Y denotes $C_1$- or $C_2$-alkylene, and

Z denotes a radical of the formula $—COOR^3$, and $R^3$ denotes $(C_1—C_8)$-alkyl, $(C_3—C_4)$-alkenyl, $(C_3—C_4)$ alkynyl or a cation.

4. A compound of the formula (I) as claimed in claim 3, in which R, $R^1$, $R^2$, $R^3$, $R^4$, $R^5$, X, Y and Z have the meanings given, with the proviso that at least one of the radicals R and $R^2$ is other than hydrogen.

5. N-Methyl-N-(6-chloro-benzothiazol-2-yl)-glycine ethyl ester.

6. N-Methyl-N-benzoazol-2-yl-glycine ethyl ester.

7. N-Methyl-N-benzothiazol-2-yl-glycine ethyl ester.

8. N-Methyl-N-benzothiazol-2-yl-glycine isobutyl ester.

9. A process for the preparation of a compound of the formula I as claimed in claim 4, which comprises

a) reacting a compound of the formula

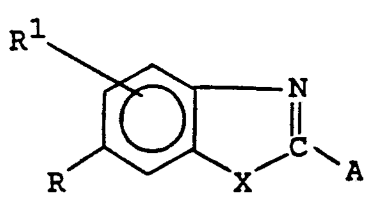

(II)

in which A denotes a group which can easily be split off ("leaving group"), with a compound of the formula

$$HN(R^2)—Y—COOR^3 \quad (III)$$

or

b) reacting a compound of the formula

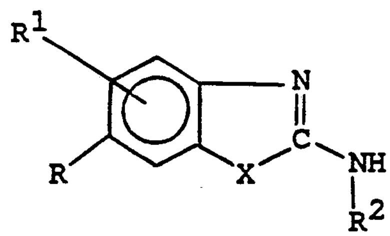

(IV)

with a compound of the formula

$$Hal—Y—COOR^3 \quad (V)$$

and, if desired, converting the resulting compound into another compound of the formula I in a manner which is known per se, for example by esterification, hydrolysis, salt formation, amidation, alkylation or dehydration.

10. A plant-protecting combination containing

a) a herbicide from the series comprising phenoxyphenoxy-, benzoxazolyloxyphenoxy-, benzothiazolyloxyphenoxy- and benzylphenoxy-carboxylic acid esters and

b) a compound of the formula I as claimed in claim 1.

11. A plant-protecting combination as claimed in claim 10, wherein the herbicide (a) is diclofop-methyl or fenoxaprop-ethyl.

12. A method of protecting useful plants from the phytotoxic side effects of herbicides, which comprises treating the plants to be protected or the cultivation area with a compound of the formula I as claimed in claim 1 before, after or at the same time as the application of a herbicide from the series comprising phenoxyphenoxy-, benzoxazolyloxyphenoxy-, benzothiazolyloxyphenoxy- and benzylphenoxy-carboxylic acid esters.

**Claims for the Contracting State: AT**

1. The use of a compound of the formula I

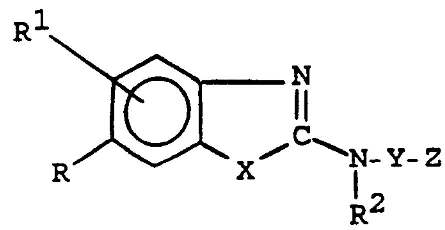

(I)

in which

X denotes O or S;

R and $R^1$ denote hydrogen, halogen, $(C_1—C_4)$-alkyl, $(C_1—C_4)$-alkoxy, CN, $NO_2$ or $CF_3$;

$R^2$ denotes hydrogen or $(C_1—C_4)$-alkyl;

Y denotes straight-chain or branched $(C_1—C_8)$-alkylene, 1—3 carbon atoms of which are in a straight chain between the amine nitrogen and the group Z; or denotes methylthio-$(C_1—C_3)$-alkylene, phenyl-$(C_1—C_3)$-alkylene or phenylene;

Z denotes

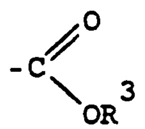 , 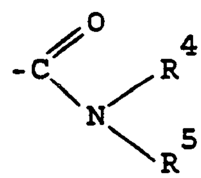 , -CN oder 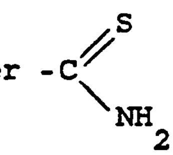 ;

$R^3$ denotes H, $(C_1—C_8)$-alkyl, which can optionally be substituted by halogen or $(C_1—C_2)$-alkoxy, $(C_3—C_4)$-alkenyl, $(C_3—C_4)$-alkynyl or the cation of an inorganic or organic base;

$R^4$ denotes H or $(C_1—C_6)$-alkyl;

$R^5$ denotes H, $(C_1—C_6)$-alkyl or phenyl, which can optionally be mono- or disubstituted by halogen, $(C_1—C_4)$-alkyl or $(C_1—C_4)$-alkoxy; or

$R^4$ and $R^5$ together with the nitrogen atom denote a pyrrolidino, piperidino or morpholino radical for protecting useful plants from the phytotoxic effects of herbicides.

2. The use as claimed in claim 1, wherein R, $R^1$, $R^2$, $R^3$, $R^4$, $R^5$, X, Y and Z have the meanings given, with the proviso that at least one of the radicals R, $R^1$ and $R^2$ is other than hydrogen.

3. The use as claimed in claim 1, wherein, in a compound of the formula I,

X denotes O or S,

R denotes H, $CH_3$ or Cl,

$R^1$ denotes H,

$R^2$ denotes H or $CH_3$,

Y denotes $C_1$- or $C_2$-alkylene, and

Z denotes a radical of the formula $—COOR^3$, and $R^3$ denotes $(C_1—C_8)$-alkyl, $(C_3—C_4)$-alkenyl, $(C_3—C_4)$ alkynyl or a cation.

4. The use as claimed in claim 3, in which R, $R^1$, $R^2$, $R^3$, $R^4$, $R^5$, X, Y and Z have the meanings given, with the proviso that at least one of the radicals R and $R^2$ is other than hydrogen.

5. The use as claimed in claim 3, wherein the compound of the formula (I) which is used is N-Methyl-N-(6-chloro-benzothiazol-2-yl)-glycine ethyl ester.

6. The use as claimed in claim 3, wherein the compound of the formula (I) is N-Methyl-N-benzoazol-2-yl-glycine ethyl ester.

7. The use as claimed in claim 3, wherein the compound of the formula (I) is N-Methyl-N-benzothiazol-2-yl-glycine ethyl ester.

8. The use as claimed in claim 3, wherein the compound of the formula is N-Methyl-N-benzothiazol-2-yl-glycine isobutyl ester.

9. A process for the preparation of a compound of the formula I as defined in claim 4, which comprises

a) reacting a compound of the formula

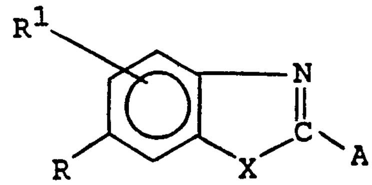

(II)

in which A denotes a group which can easily be split off ("leaving group"), with a compound of the formula

$$\underset{\displaystyle R^2}{\overset{}{HN}}—Y—COOR^3 \qquad (III)$$

or

b) reacting a compound of the formula

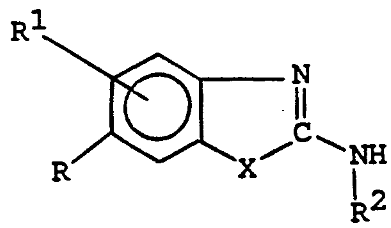

(IV)

with a compound of the formula

$$Hal—Y—COOR^3 \qquad (V)$$

and, if desired, converting the resulting compound into another compound of the formula I in a manner which is known per se, for example by esterification, hydrolysis, salt formation, amidation, alkylation or dehydration.

10. A plant-protecting combination containing

a) a herbicide from the series comprising phenoxyphenoxy-, benzoxazolyloxyphenoxy-, benzothiazolyloxyphenoxy- and benzylphenoxy-carboxylic acid esters and

b) a compound of the formula I as claimed in claim 1.

11. A plant-protecting combination as claimed in claim 10, wherein the herbicide (a) is diclofop-methyl or fenoxaprop-ethyl.

12. A method of protecting useful plants from the phytotoxic side effects of herbicides, which comprises treating the plants to be protected or the cultivation area with a compound of the formula I as claimed in claim 1 before, after or at the same time as the application of a herbicide from the series comprising phenoxyphenoxy-, benzoxazolyloxyphenoxy-, benzothiazolyloxyphenoxy- and benzylphenoxy-carboxylic acid esters.